# EUROPEAN PATENT SPECIFICATION

(11) **EP 4 470 531 B1**
(45) Date of publication and mention of the grant of the patent: **02.12.2026**
(21) Application number: 23747041.4
(22) Date of filing: 26.01.2023
(51) Int. Cl.: A61K 9/51, A61K 9/00, A61K 9/14, A61K 31/47, A61P 27/02

(54) **PHARMACEUTICAL COMPOSITION FOR TREATING RETINITIS PIGMENTOSA**
PHARMAZEUTISCHE ZUSAMMENSETZUNG ZUR BEHANDLUNG VON RETINITIS PIGMENTOSA
COMPOSITION PHARMACEUTIQUE POUR LE TRAITEMENT DE LA RÉTINITE PIGMENTAIRE

(30) Priority: 28.01.2022 JP 2022012146
(43) Date of publication of application: 04.12.2024
(73) Proprietor: Kyushu University, National University Corporation, Nishi-ku Fukuoka-shi Fukuoka 819-0395 (JP); SENTAN Pharma Inc., Fukuoka-shi Fukuoka 812-0027 (JP)
(72) Inventor: MURAKAMI, Yusuke, Fukuoka-shi, Fukuoka 819-0395 (JP); SONODA, Koh-Hei, Fukuoka-shi, Fukuoka 819-0395 (JP); IKEDA, Yasuhiro, Fukuoka-shi, Fukuoka 819-0395 (JP); KOGA, Tsuneyuki, Kurume-shi, Fukuoka 839-0864 (JP); MATSUO, Takeru, Kurume-shi, Fukuoka 839-0864 (JP); EGUCHI, Yojiro, Kurume-shi, Fukuoka 839-0864 (JP); FURUIE, Hironobu, Kurume-shi, Fukuoka 839-0864 (JP)
(74) Representative: Vossius & Partner Patentanwälte Rechtsanwälte mbB
(86) International application number: PCT/JP2023/002470
(87) International publication number: WO 2023/145831

(56) References cited:
- WO-A1-2008/026702
- WO-A1-2008/044339
- WO-A1-2021/180867
- WO-A2-2004/103960
- JP-A- 2004 149 480
- US-A1- 2010 285 083
- K. KOMEIMA ET AL: "Antioxidants reduce cone cell death in a model of retinitis pigmentosa", PROCEEDINGS OF THE NATIONAL ACADEMY OF SCIENCES (PNAS), vol. 103, no. 30, 25 July 2006 (2006-07-25), pages 11300 - 11305, XP055440031, ISSN: 0027-8424, DOI: 10.1073/pnas.0604056103
- ARAFA MONA G ET AL: "Chitosan-Coated PLGA Nanoparticles for Enhanced Ocular Anti-Inflammatory Efficacy of Atorvastatin Calcium", vol. Volume 15, 28 February 2020 (2020-02-28), pages 1335 - 1347, XP093041482, Retrieved from the Internet <URL:https://www.ncbi.nlm.nih.gov/pmc/articles/PMC7053815/pdf/ijn-15-1335.pdf> DOI: 10.2147/IJN.S237314
- YADAV MONIKA ET AL: "Atorvastatin-loaded solid lipid nanoparticles as eye drops: proposed treatment option for age-related macular degeneration (AMD)", DRUG DELIVERY AND TRANSLATIONAL RESEARCH, SPRINGER, GERMANY, vol. 10, no. 4, 8 April 2020 (2020-04-08), pages 919 - 944, XP038195616, ISSN: 2190-393X, [retrieved on 20200408], DOI: 10.1007/S13346-020-00733-4
- ONO MAYUMI, JUN-ICHI FUKUSHI, DAISUKE GOTO, HITOE TORISU, SHIGEO YOSHIDA, MICHIHIKO KUWANO: "Mechanism of Angiogenesis and Angiogenic Diseases", KAGAKU TO SEIBUTSU, vol. 37, no. 1, 1 January 1999 (1999-01-01), pages 14 - 19, XP093081057

## Description

### TECHNICAL FIELD

This application claims priority to Japanese Patent Application No. 2022-12146.

The present disclosure relates to treatment of retinitis pigmentosa.

### BACKGROUND ART

Retinitis pigmentosa is a disease in which photoreceptor cells and pigment epithelial cells of the retina degenerate due to genetic mutations. Its typical symptoms include night blindness and visual field constriction due to photoreceptor cell damage. The frequency of retinitis pigmentosa is considered one in 4,000 to 8,000 people. Currently, there is no established treatment that restores retinal function or inhibits disease progression and only symptomatic treatment is available. Komeima et al., 103(30), 11300-11305 (2006) discloses the treatment of retinitis pigmentosa using a mixture of antioxidants, including α-tocopherol, ascorbic acid, Mn-derived porphyrin, and α-lipoic acid, which reduces cone cell death when administered intravenously.

Statins lower blood cholesterol by inhibiting HMG-CoA reductase and are widely used as therapeutic agents for hypercholesterolemia. Statins are also known to have pro-angiogenic effect. This effect was initially observed in animal models only at doses much higher than the clinical dose. Then, it was reported that pitavastatin-loaded PLGA nanoparticles were selectively delivered to vascular endothelial cells and induced angiogenesis at low doses in a lower limb ischemia model, and that they improved symptoms of pulmonary hypertension at low doses in a pulmonary hypertension model. Clinical trials are now underway for the treatment of critical limb ischemia and pulmonary hypertension, respectively. Pitavastatin-loaded PLGA nanoparticles have also been reported to inhibit tissue damage in an atherosclerosis model.

### PRIOR ART DOCUMENT

### PATENT DOCUMENT

Patent Document 1: WO2008/026702
Non-patent Document 1: K. Ichimura et al., Int Heart J 59, 1432-1444 (2018).
Non-patent Document 2: S. Katsuki et al., Circulation 129, 896-906 (2014).

### SUMMARY OF THE INVENTION

### PROBLEMS TO BE SOLVED BY THE INVENTION

An object of the present disclosure is to provide a pharmaceutical composition for treating retinitis pigmentosa. The present invention is as defined in the appended claims.

### SOLUTIONS TO THE PROBLEMS

The inventors found that inflammatory monocytes in the peripheral blood and peripherally-derived macrophages in the retina increased in retinitis pigmentosa, and that statin-loaded nanoparticles reduced peripheral blood inflammatory monocytes and peripherally-derived retinal macrophages and inhibited cone cell death in an animal model of retinitis pigmentosa, and then accomplish the present invention.

In an aspect, the present disclosure provides a pharmaceutical composition for treating retinitis pigmentosa comprising statin-loaded nanoparticles.

### EFFECTS OF THE INVENTION

The present disclosure provides a pharmaceutical composition for treating retinitis pigmentosa comprising statin-loaded nanoparticles.

### BRIEF DESCRIPTION OF THE DRAWINGS

[Fig. 1] Fig. 1 shows the proportions of inflammatory monocytes (IMo) in the wild type (WT) and rd10 mice (postnatal day 21 (P21): WT n=7, rd10 n=7; P31: WT n=8, rd10 n=6; P42: WT n=5, rd10 n=5). The central horizontal lines indicate the medians, boxes indicate 25th to 75th percentiles, and whiskers indicate 1.5 times the inter quartile range from the bottom and the top of the box. Outliers are shown as dots. *P<0.05, **P<0.01 from Wilcoxon rank sum tests. (Following figures are shown in the same manner.)
[Fig. 2] Fig. 2 shows the percentage of monocyte subsets in the retinitis pigmentosa (RP) patients (n=31) and controls (n=16).
[Fig. 3] Fig. 3 shows the correlation between the percentage of monocyte subsets and the decline rate of retinal sensitivity (MD slope) in the retinitis pigmentosa (RP) patients.
[Fig. 4] Fig. 4 shows the percentage of microglia (P21: WT n=8, rd10 n=8; P31: WT n=9, rd10 n=6; P42: WT n=7, rd10 n=9) (left) and macrophages (P21: WT n=8, rd10 n=8; P31: WT n=9, rd10 n=6; P42: WT n=7, rd10 n=9) (right) in live cells of WT and rd10 mouse retina.
[Fig. 5] Fig. 5 shows the percentage of IMo in the peripheral blood of rd10 mice and rd10;Ccl2^{-/-} mice (P21: rd10 n=6, rd10;Ccl2^{-/-} n=5; P31: rd10 n=7, rd10;Ccl2^{-/-} n=7; P42: rd10 n=5, rd10;Ccl2^{-/-} n=7).
[Fig. 6] Fig. 6 shows the percentage of resident microglia (left) and macrophages (right) in live cells of rd10 mice and rd10;Ccl2^{-/-} mice (P21: rd10 n=6, rd10;Ccl2^{-/-} n=6; at P31: rd10 n=5, rd10;Ccl2^{-/-} n=5; and at P42: rd10 n=5, rd10;Ccl2^{-/-} n=5).
[Fig. 7] Fig. 7 shows TUNEL staining (left) of the retina and the number of TUNEL-positive cells in the central, mid and peripheral areas of nasal and temporal hemispheres (rd10 n=8, rd10;Ccl2^{-/-} n=8) (right) in the retina of rd10 (rd10;Ccl2^{+/+}) mice and rd10;Ccl2^{-/-} mice (P21). Scale bar = 50 µm.
[Fig. 8] Fig. 8 shows hematoxylin and eosin (HE) staining (left) of the retina and the number of photoreceptor cells in the central, mid and peripheral areas of nasal and temporal hemispheres (rd10 n=20, rd10;Ccl2^{-/-} n=10) (right) in the retina of rd10 (rd10;Ccl2^{+/+}) mice and rd10;Ccl2^{-/-} mice (P26). Scale bar = 50 µm.
[Fig. 9] Fig. 9 shows peanut agglutinin (PNA) staining (left) and the number of PNA-positive cone cells in the areas located 200 µm and 500 µm from the optic disc (rd10 n=21, rd10;Ccl2^{-/-} n=15) (right) of rd10 (rd10;Ccl2^{+/+}) mice and rd10;Ccl2^{-/-} mice (P42). Scale bar = 50 µm.
[Fig. 10] Fig. 10 shows the photopic electroretinogram (ERG) (light) and ERG b-wave amplitude (rd10 n=6, rd10;Ccl2^{-/-} n=8) (right) of rd10 (rd10;Ccl2^{+/+}) mice and rd10;Ccl2^{-/-} mice (P35).
[Fig. 11] Fig. 11 shows fluorescein isothiocyanate (FITC) incorporation in peripheral blood IMo of rd10 mice (P17) that intravenously received PBS or FITC-loaded nanoparticles (FITC-NP) (PBS n=4; FITC-NP n=4). Left shows representative data. Right shows the result of quantification (the percentage above threshold)
[Fig. 12] Fig. 12 shows FITC incorporation in the retina samples of rd10 mice (P17) that intravenously received PBS or FITC-NP (PBS n=4; FITC-NP n=4). Left shows the representative data of microglia and macrophages. Right shows the result of quantification (the percentage above threshold).
[Fig. 13] Fig. 13 shows the percentage of peripheral blood IMo (PBS n=10; FITC-NP n=11; PVS-NP; n=14) (upper) and the percentage of macrophages and microglia in live cells of the retina (PBS n=14; FITC-NP n=12; PVS-NP; n=14) (lower) of rd10 mice (P31) that intravenously received PBS, FITC-NP, or pitavastatin-loaded nanoparticles (PVS-NP).
[Fig. 14] Fig. 14 shows PNA staining of the retina (left) and the number of PNA-positive cone cells in the areas located 200 µm and 500 µm from the optic disc (PBS n=9; FITC-NP n=10; PVS-NP; n=9) (right) of rb10 mice (P52) that intravenously received PBS or PVS-NP. Scale bar = 50 µm.
[Fig. 15] Fig. 15 shows the photopic ERG (left) and ERG b-wave amplitude (PBS n=6; PVS-NP n=8) (right) of rb10 mice (P35) that intravenously received PBS or PVS-NP.
[Fig. 16] Fig. 16 shows the photopic ERG b-wave amplitude of rb10 mice (P49) that intravenously received PBS (100µl PBS), PVS-NP low (0.1 mg PVS/kg), PVS-NP middle (0.3 mg PVS/kg), or PVS-NP high (1.0 mg PVS/kg) (PBS n=8; PVS-NP low n=10; PVS-NP middle n=20; PVS-NP high n=20).
[Fig. 17] Fig. 17 shows the number of PNA-positive cone cells of rb10 mice (P49) that intravenously received PBS (100µl PBS), PVS-NP low (0.1 mg PVS/kg), PVS-NP middle (0.3 mg PVS/kg), or PVS-NP high (1.0 mg PVS/kg) (PBS n=5; PVS-NP low n=6; PVS-NP middle n=7; PVS-NP high n=8). Left and right show the results in the areas located 250 µm and 750 µm from the optic disc, respectively.
[Fig. 18] Fig. 18 shows the mean of the numbers of PNA-positive cone cells in the two areas of Fig. 17 for each mouse.
[Fig. 19] Fig. 19 shows the number of PNA-positive cells (PBS 2/M n=15; PVS-NP 1/M n=14; PVS-NP 2/M n=16) (left) and photopic ERG b-wave amplitude (PBS 2/M n=24; PVS-NP 1/M n=14; PVS-NP 2/M n=16) (right) of rb10 mice (P49) that intravenously received PBS (100 µl PBS) every two weeks (PBS 21M) or PVS-NP (0.75 mg PVS/kg) every four weeks (PVS-NP 1/M) or PVS-NP (0.5 mg PVS/kg) every two weeks (PVS-NP 2/M).

### DETAILED DESCRIPTION

Unless otherwise specified, terms used herein have the meanings as generally understood by those skilled in the art in the fields such as organic chemistry, medical science, pharmaceutical science, molecular biology, and microbiology. The followings are definitions for some terms used herein, which definitions herein take precedence over the general understandings.

In an aspect, the present disclosure provides a pharmaceutical composition for treating retinitis pigmentosa comprising statin-loaded nanoparticles.

As used herein, the term statin refers to a compound having HMG-CoA (3-hydroxy-3-methylglutaryl-coenzyme A) reductase inhibitory activity. Statin includes, for example, pitavastatin, atorvastatin, pravastatin, simvastatin, fluvastatin, and rosuvastatin. In one embodiment, the statin is pitavastatin. The term statin referred in the present disclosure is used in the sense that it encompasses the free form and its pharmaceutically acceptable salts, and their solvates. Examples of pharmaceutically acceptable salts include alkali metal salts such as sodium and potassium salts, alkaline earth metal salts such as calcium and magnesium salts, organic amine salts such as phenethylamine salts, and ammonium salts. Examples of solvates include solvents with water or alcohol. For example, pharmaceutically acceptable salts of pitavastatin includes pitavastatin calcium, and solvates of pitavastatin or a pharmaceutically acceptable salt thereof include pitavastatin calcium hydrate (for example, pentahydrate). The statin-loaded nanoparticles may comprise one or more statins, and may comprise any other agent than the statin(s).

The statin-loaded nanoparticles of the present disclosure contain statin inside a nanoparticle composed of a biocompatible polymer. The biocompatible polymer may be a polymer of one or more monomers selected from D,L-lactide, D-lactide, L-lactide, D,L-lactic acid, D-lactic acid, L-lactic acid, glycolide, glycolic acid, ε-caprolactone, ε-hydroxyhexanoic acid, γ-butyrolactone, γ-hydroxybutyric acid, δ-valerolactone, δ-hydroxyvaleric acid, hydroxybutyric acid, and malic acid. Examples of biocompatible polymers include, for example, polylactic acid, polyglycolic acid, lactic acid-glycolic acid copolymer (also called as poly lactide-co-glycolide, PLGA), and lactic acid-aspartic acid copolymer. In one embodiment, the biocompatible polymer is PLGA.

The term PLGA includes polymers containing lactic acid or lactide and glycolic acid or glycolide in various proportions. The ratio of lactic acid or lactide to glycolic acid or glycolide may be, for example, 1:99 to 99:1, preferably 3:1. The weight-average molecular weight of PLGA may be, for example, 5,000 to 200,000 or 15,000 to 25,000. PLGA can be synthesized by any known process. Commercially available PLGA may also be used.

The particle size of statin-loaded nanoparticles may be, for example, 1 to 1000 nm, 2 to 500 nm, 3 to 300 nm, 10 to 300 nm, or 50 to 300 nm. The particle size may also be 100 to 300 nm or 200 to 300 nm. In one embodiment, the particle size is 50 to 300 nm. The particle size as used herein refers to the sphere equivalent diameter measured by dynamic light scattering and is expressed as a median diameter (D₅₀). The median diameter (D₅₀) is the particle diameter at the point that is 50% in the cumulative curve where the total volume of the population of particles is 100% (50% diameter). The cumulative curve and D₅₀ can be determined using a particle size analyzer such as Nanotrac Wave-EX150 (MictotracBEL Corp.).

The surface of the statin-loaded nanoparticles may be modified with polyethylene glycol (PEG). For example, nanoparticles whose surface is modified with PEG can be obtained by using a PEG-modified biocompatible polymer for the production of nanoparticles. The modification of the particle surface with PEG can improve the stability of nanoparticles in blood.

The statin-loaded nanoparticles can be produced by any production process. For example, the statin-loaded nanoparticles can be produced by the water-emulsion method. The water-emulsion method uses two types of solvents: a good solvent in which the biocompatible polymer is dissolved, and a poor solvent in which the biocompatible polymer is not dissolved. The good solvent and the poor solvent can be selected by a person skilled in the art according to the nanoparticles to be produced.

Examples of poor solvents include water. When water is used as the poor solvent, a surfactant may be added to the water. Examples of surfactants include, for example, polyvinyl alcohol (PVA), lecithin, hydroxymethylcellulose, and hydroxypropylcellulose. In one embodiment, the poor solvent is water and the surfactant is PVA.

Examples of good solvents include haloalkanes, acetone, methanol, ethanol, ethyl acetate, diethyl ether, cyclohexane, benzene, toluene, and mixtures of these organic solvents, which have a low boiling point and are water insoluble. In one embodiment, the good solvent is acetone or a mixture of acetone and ethanol at a ratio of 2:1.

In the water-emulsion method, a biocompatible polymer is first dissolved in a good solvent, and a solution containing an agent is then added to and mixed with the good solvent. When the good solvent containing the polymer and agent is dropped into a poor solvent under agitation, the good solvent diffuses into the poor solvent rapidly. As a result, emulsification of the good solvent in the poor solvent occurs to form emulsion drops of the good solvent. Then, due to interdiffusion between the good and poor solvents, the organic solvent continuously diffuses from inside of the emulsion into the poor solvent, reducing the solubility of the biocompatible polymer and agent in the emulsion drops and producing nanoparticles of spherical crystalline particles that encapsulate the agent. The good solvent, an organic solvent, is then separated by centrifugation or removed under reduced pressure. The resulting nanoparticles can be used *per se* or after being powdered by lyophilization or other operations.

The statin-loaded nanoparticles may be produced by using a forced-thin-film microreactor. When the forced thin-film microreactor is used, a good solvent containing the polymer and agent and a poor solvent are first introduced between the processing planes, which are arranged facing each other, and at least one of which rotates relative to the other. The good solvent is mixed with the poor solvent in the thin-film fluid formed by this process, and nanoparticles encapsulating the agent are deposited in the thin-film fluid. The forced thin-film microreactor may be ULREA SS-11 (M Technique Co., Ltd.), for example.

The statin-loaded nanoparticles may contain, for example, 0.01 to 99% by weight, 0.1 to 30% by weight, 0.5 to 20% by weight, or 1 to 15% by weight of statin. In one embodiment, the statin-loaded nanoparticles contain 1 to 15% by weight of statin. In the present disclosure, the statin content is expressed as a ratio of the weight of statin to the weight of statin-loaded nanoparticles. The statin content can be determined by measuring the weight of statin extracted from a given weight of statin-loaded nanoparticles and calculating the ratio of the weight of statin to the weight of statin-loaded nanoparticles.

The statin-loaded nanoparticles can be complexed into redispersible aggregates (nanocomposites) when powdered by lyophilization or other operations. For example, nanoparticles can be prepared as redispersible composites when they are dried with organic or inorganic material. This compositing allows nanoparticles to be aggregates that are easy to handle and can disperse and exhibit their properties when exposed to moisture at the time of use. In one embodiment, the statin-loaded nanoparticles are composites with sugar alcohol or sucrose. The use of a substance such as sugar alcohol can reduce the variability of the inclusion rate, and this substance can also serve as an excipient to enhance the handling of nanoparticles. Examples of sugar alcohol include mannitol, trehalose, sorbitol, erythritol, maltitol, and xylitol. In one embodiment, the sugar alcohol is trehalose. Instead of lyophilization, the compositing can be done by fluid bed dry granulation.

The examples of the present disclosure show that inflammatory monocytes in the peripheral blood and peripherally-derived macrophages in the retina increased in retinitis pigmentosa, and that statin-loaded nanoparticles reduced peripheral blood inflammatory monocytes and peripherally-derived retinal macrophages and inhibited cone cell death in an animal model of retinitis pigmentosa. Therefore, the statin-loaded nanoparticles can be used for the treatment of retinitis pigmentosa.

Retinitis pigmentosa is a disease in which photoreceptor cells and pigment epithelial cells of the retina degenerate due to genetic mutations. Retinitis pigmentosa includes conditions in which any one of rod cells, cone cells, and pigment epithelial cells, or two or more cells selected from these cells, degenerate. In retinitis pigmentosa, rod cell degeneration is often preceded by gradual cone cell degeneration. The condition in which only rod cells degenerate is called rod dystrophy, and the condition in which both rod cells and cone cells degenerate is called rod-cone dystrophy, both of which are included in retinitis pigmentosa of the present disclosure. Retinitis pigmentosa may be caused by any genetic mutation, and there may be one or more causative genetic mutations.

As used herein, the treatment of retinitis pigmentosa includes improvement, inhibition and delay of exacerbation of one or more symptoms or findings of retinitis pigmentosa, and inhibition and delay of disease progression. Symptoms of retinitis pigmentosa include night blindness, visual field constriction, visual acuity loss, photophobia, day blindness, color blindness, and photopsia. Findings of retinitis pigmentosa include (1) fundus findings (retinal vascular narrowing, coarse retinal pigmentation, ossicle-like pigmentation, multiple white spots, optic nerve atrophy, macular degeneration), (2) abnormal electroretinograms (weakening type, negative type, vanishing type), (3) fundus autofluorescence findings of hyperfluorescence or hypofluorescence due to retinal pigment epithelial atrophy, and (4) optical coherence tomography findings of abnormal ellipsoid zones (IS/OS) in the central fossa (discontinuity or disappearance).

The statin-loaded nanoparticles can be comprised in a pharmaceutical composition. The pharmaceutical composition may comprise, for example, 0.000001 to 99.9% by weight, 0.00001 to 99.8% by weight, 0.0001 to 99.7% by weight, 0.001 to 99.6% by weight, 0.01 to 99.5% by weight, 0.1 to 99% by weight, 1 to 50% by weight, 1 to 40% by weight, 1 to 30% by weight, 1 to 20% by weight, or 1 to 15% by weight of statin-loaded nanoparticles. The pharmaceutical composition may further comprise a pharmaceutically acceptable additive as needed. Examples of pharmaceutical acceptable additives include, for example, excipients, lubricants, binders, disintegrants, solubilizers, suspending agents, isotonic agents, buffers, painless agents, preservatives, antioxidants, coloring agents, and sweetening agents.

The pharmaceutical composition may be, for example, in the form of tablets, capsules, dispersions, granules, liquids, suspensions, emulsions, inhalants, injections, eye drops, and ophthalmic ointments. Examples of injections include solution injections, suspension injections, emulsion injections, and ready-to-use injections. In one embodiment, the pharmaceutical composition is an injection. These formulations can be prepared by conventional processes.

The statin-loaded nanoparticles or the pharmaceutical composition comprising the statin-loaded nanoparticles is administered to a subject in an amount that can exert the desired effect (herein referred to as an effective amount). The dosage and duration of administration may be determined by a person skilled in the art according to factors such as age, weight, and health condition of the subject. The statin-loaded nanoparticles or the pharmaceutical composition comprising the statin-loaded nanoparticles may be administered, for example, at a dose of 0.001 mg/kg to 100 mg/kg, 0.003 mg/kg to 10 mg/kg, 0.005 mg/kg to 5 mg/kg, 0.01 mg/kg to 3 mg/kg, 0.01 mg/kg to 1 mg/kg, 0.01 mg/kg to 0.75 mg/kg, 0.01 mg/kg to 0.5 mg/kg, 0.01 mg/kg to 0.3mg/kg, 0.01 mg/kg to 0.25 mg/kg, 0.01 mg/kg to 0.1 mg/kg, 0.01 mg/kg to 0.09 mg/kg, 0.01 mg/kg to 0.08 mg/kg, 0.01 mg/kg to 0.07 mg/kg, 0.01 mg/kg to 0.06 mg/kg, 0.01 mg/kg to 0.05 mg/kg, 0.01 mg/kg to 0.04 mg/kg, 0.01 mg/kg to 0.03 mg/kg, 0.03 mg/kg to 1 mg/kg, 0.03 mg/kg to 0.75 mg/kg, 0.03 mg/kg to 0.5 mg/kg, 0.03 mg/kg to 0.3 mg/kg, 0.03 mg/kg to 0.25 mg/kg, 0.03 mg/kg to 0.1 mg/kg, 0.03 mg/kg to 0.09 mg/kg, 0.03 mg/kg to 0.08 mg/kg, 0.03 mg/kg to 0.07 mg/kg, 0.03 mg/kg to 0.06 mg/kg, 0.03 mg/kg to 0.05 mg/kg, 0.1 mg/kg to 1 mg/kg, 0.1 mg/kg to 0.75 mg/kg, 0.1 mg/kg to 0.5 mg/kg, 0.1 mg/kg to 0.3 mg/kg, 0.3 mg/kg to 1 mg/kg, 0.3 mg/kg to 0.75 mg/kg, or 0.3 mg/kg to 0.5 mg/kg of statin per day, which can be administered in a single dose or in multiple divided doses (for example, 2, 3 or 4 doses). In one embodiment, the statin-loaded nanoparticles or the pharmaceutical composition comprising the statin-loaded nanoparticles is administered at a dose of 0.01 mg/kg to 0.3 mg/kg or 0.03 mg/kg to 0.1 mg/kg of statin (for example, pitavastatin calcium) per day. The statin-loaded nanoparticles or the pharmaceutical composition comprising the statin-loaded nanoparticles may be administered at a dose of 1 to 10 mg/body (for example, 1, 2, 4, 8, or 10 mg/body) or 1 to 8 mg/body (for example, 1, 2, 4, or 8 mg/body) of statin (for example, pitavastatin calcium) per adult per day. The administration may be in a single dose or multiple doses. For multiple doses, the administration may be done once a day, once every several days (for example, 2, 3, 4, 5 or 6 days), once a week or once every several weeks (for example, 2, 3, 4, 5 or 6 weeks), once a month or once every several months (for example, 2, 3, 4, 5 or 6 months). In one embodiment, the administration is twice a week (for example, once every 3 or 4 days) or once every 1 to 4 weeks (for example, 1, 2, 3, or 4 weeks). The duration of administration may be, for example, one day or several days (for example, 2, 3, 4, 5 or 6 days), one week or several weeks (for example, 2, 3, 4, 5 or 6 weeks), one month or several months (for example, 2, 3, 4, 5 or 6 months), or longer. The administration may be systemic or topical, and may be oral or parenteral (for example, intravenous, intramuscular, intratracheal, intranasal, intraocular). In one embodiment, the statin-loaded nanoparticles or the pharmaceutical composition comprising the statin-loaded nanoparticles is administered intravenously.

In the present disclosure, the subject is a mammal (for example, human, mouse, rat, hamster, rabbit, cat, dog, cow, sheep, monkey), preferably a human. In one embodiment, the subject is a human subject suffering from retinitis pigmentosa (also referred to as a retinitis pigmentosa patient).

In an aspect, the present disclosure provides a method for treating retinitis pigmentosa, comprising administering an effective amount of statin-loaded nanoparticles to a subject in need thereof.

In an aspect, the present disclosure provides statin-loaded nanoparticles for treating retinitis pigmentosa.

In an aspect, the present disclosure provides use of statin-loaded nanoparticles for the manufacture of a medicament for treating retinitis pigmentosa.

In an aspect, the present disclosure provides use of statin-loaded nanoparticles for treating retinitis pigmentosa.

Exemplary embodiments of the present disclosure are described below.
[1] A pharmaceutical composition for treating retinitis pigmentosa comprising statin-loaded nanoparticles.
[2] The pharmaceutical composition according to item 1, wherein the statin is pitavastatin or a pharmaceutically acceptable salt thereof.
[3] The pharmaceutical composition according to items 1 or 2, wherein the statin is pitavastatin calcium.
[4] The pharmaceutical composition according to any one of items 1 to 3, wherein the statin-loaded nanoparticles contain 1 to 15% by weight of statin.
[5] The pharmaceutical composition according to any one of items 1 to 4, wherein the statin-loaded nanoparticles contain PLGA.
[6] The pharmaceutical composition according to any one of items 1 to 5, wherein the statin-loaded nanoparticles have a particle size of 50 to 300 nm.
[7] The pharmaceutical composition according to any one of items 1 to 6, wherein the pharmaceutical composition comprises 1 to 15% by weight of the statin-loaded nanoparticles.
[8] The pharmaceutical composition according to any one of items 1 to 7, wherein the pharmaceutical composition is administered at a dose of 0.01 mg/kg to 0.5 mg/kg per day of statin.
[9] The pharmaceutical composition according to any one of items 1 to 8, wherein the pharmaceutical composition is administered intravenously.
[10] The pharmaceutical composition according to any one of items 1 to 9, wherein the pharmaceutical composition is administered twice a week or once every 1 to 4 weeks.
[11] A method for treating retinitis pigmentosa, comprising administering an effective amount of statin-loaded nanoparticles to a subject in need thereof.
[12] Use of statin-loaded nanoparticles for the manufacture of a medicament for treating retinitis pigmentosa.
[13] Use of statin-loaded nanoparticles for treating retinitis pigmentosa.

The present invention will be described in more detail by referring to examples below.

### EXAMPLES

### 1. Materials and Methods

### Animals

WT (C57BL/6J) mice, B6.CXB1-Pde6β^{rd10}/J (rd10) mice, and B6.129S4-Ccl2^{tm1Rol}/J (Ccl2^{-/-}) mice were purchased from The Jackson Laboratory (West Grove, PA). Rd10 mice were crossed with Ccl2^{-/-} mice to generate rd10;Ccl2^{-/-} mice.

### Patients and Control subjects

Because blood monocytes could be affected by systemic disorders such as cardiovascular diseases, hypertension, and diabetes, we included systemically healthy and relatively young subjects <45 years old. Retinitis pigmentosa (RP) patients were diagnosed based on the patient's history of night blindness, characteristic fundus findings (e.g., bone spicule-like pigment clumping in the mid-peripheral and peripheral retina), visual field constriction and/or ring scotoma, and reduced or non-recordable a- and b-wave amplitude on electroretinography. Patients with cone dystrophy, cone-rod dystrophy, Bietti crystalline retinopathy, uveitis, or systemic diseases were excluded. Participants were consecutively recruited in 2017 and 2018, and blood samples were collected from 31 RP patients and 16 age- and gender-matched healthy subjects. The RP patients were followed up >1 year and underwent HFA10-2 tests at least three times to obtain the mean deviation (MD) slope. For the analysis, the subject's visual acuity at the time of blood collection, the HFA10-2 test result at the closest time to the blood collection, and the MD slope were used. The examination results of the right eye of each subject were used for analysis.

### Clinical examinations

The subjects' best corrected visual acuity (BCVA) was measured with a Landolt decimal VA chart (CV-6000; Tomey, Nagoya, Japan) at 5 m or with single Landolt test cards (HP-1258; Handaya, Tokyo), and the values were converted to the logarithm of the minimum angle of resolution (logMAR) units. The refractive error was corrected at each visit using multiples lenses with different diopters to confirm that the VA was best-corrected. The VA was based on the minimum Landolt C letter that the subject was able to correctly answer >60% (3/5) of the time. Automated static perimetry tests were performed with a Humphrey Field Analyzer (HFA; Humphrey Instruments, San Leandro, CA) using the central 10-2 Swedish Interactive Thresholding Algorithm Standard Program. The lens was corrected as appropriate for the test distance. Visual field testing was repeated if the test reliability was not satisfactory (i.e., fixation loss >20%, false positive >15%, or false negative >33%).

### Flow cytometry

Immunolabeled cells were analyzed by the BD FACSVerse system (BD Biosciences, Franklin Lakes, NJ) using FlowJo software. The samples were prepared and assayed as follows.

### - Mouse blood monocytes

Peripheral blood was drawn from the mouse via a cardiac puncture, and red blood cells were lysed with VersaLyse Lysing solution (Becton Dickinson Biosciences, San Jose, CA) for 10 min at room temperature. The cells were washed twice in ice-cold FACS buffer, i.e., phosphate buffered saline (PBS) with 2% fetal bovine serum (FBS). Fc receptors were blocked with anti-mouse CD16/CD32 (eBioscience) for 5 min at 4°C, followed by incubation for 20 min on ice with antibodies against mouse CD192 (CCR2) (Alexa Fluor 647-conjugated, clone SA203G11; Biolegend, San Diego, CA), CD11b (BV421-conjugated, clone M1/70; Biolegend), Ly-6C (Alexa fluor 488-conjugated, clone HK1.4; Biolegend), Ly-6G (allophycocyanin [APC]-cy7-conjugated, clone 1A8; Biolegend) and CX3CR1 (phycoerythrin [PE]-conjugated, clone SA011F11, Biolegend). Dead cells were excluded with the fluorescent marker 7-AAD (BD Pharmingen, San Diego, CA). Inflammatory monocytes were identified as CD11b⁺Ly-6C^{hi}Ly-6G^{lo-neg} cells.

### - Mouse microglia and macrophages

The mouse retina was harvested from enucleated eyes, minced and digested (with 1.2 mg/ml collagenase D [Roche Diagnostics, Indianapolis, IN] and 40 µg/ml DNase I [Sigma-Aldrich, St. Louis, MO]) in a water bath at 37°C for 30 min. Following digestion, the tissue was dissociated into single-cell suspensions by pipetting. The cells were washed twice in ice-cold FACS buffer (PBS with 2% FBS). Fc receptors were blocked with anti-mouse CD16/CD32 (eBioscience) for 10 min at 4°C, followed by incubation for 20 min on ice with antibodies against mouse CD11c (PE-Cy7-conjugated, clone N418; Biolegend), CD45 (APC-conjugated, clone 30-F11; Biolegend), Ly-6C (APC-cy7-conjugated, clone HK1.4; Biolegend), Ly-6G (APC-cy7-conjugated, clone 1A8; Biolegend), CD11b (PE-conjugated, clone M1/70; Biolegend), CD192 (CCR2) (FITC-conjugated, clone SA203G11; Biolegend), and CX3CR1 (BV421-conjugated, clone SA011F11; Biolegend). Dead cells were excluded with 7-AAD (BD Pharmingen). Microglia were defined as CD11b^{hi}CD11^{mid}CD45^{mid}Ly-6G^{lo}Ly-6C^{lo}cells, and macrophages were defined as CD11b^{hi}CD11c^{hi}CD45^{hi}Ly-6G^{lo}Ly-6C^{lo}cells. WT retinas were used as a control for gating microglia and macrophages at each experiment.

### - Human blood monocytes

Whole blood (8 ml) was obtained from an antecubital vein of each subject by using a BD Vacutainer CPT Cell Preparation Tube with Sodium Heparin (BD Biosciences). Once obtained, the samples underwent immediate (within <30 min) density gradient separation of mononuclear cells. The separated mononuclear cells were then pelleted with low-speed centrifugation (200 g) and aliquoted into 5 ml tubes in PBS with 2% FBS. The samples were stored at -80°C in a freezer until analyzed, and thawed 30 min before the analyses. For immunolabeling, cells were washed twice in ice-cold FACS buffer (PBS with 2% FBS). Fc receptors were blocked with anti-mouse CD16/CD32 (eBioscience) for 5 min at 4°C, followed by incubation for 20 min on ice with antibodies against human CD56 (NCAM) (PerCP/Cy5.5-conjugated, clone HCD56; Biolegend), CD19 (PerCP/Cy5.5-conjugated, clone HIB19; Biolegend), HLA-DR (APC-conjugated, clone L243; Biolegend), CD14 (PE-conjugated, clone M5E2; Biolegend), CD16 (BV421-conjugated, clone 3G8; Biolegend), CD192 (CCR2) (FITC-conjugated, clone K036C2; Biolegend), and CX3CR1 (PEcy7-conjugated, clone 2A9-1, Biolegend). Dead cells were excluded with 7-AAD (BD Pharmingen). Monocytes were identified as HLA-DR⁺CD2⁻CD19⁻CD56⁻ cells. These cells were classified into three subsets according to the levels of CD14 and CD16 expression.

### Retinal whole-mount staining

Mouse eyes were enucleated and fixed with 4% paraformaldehyde for 1 hr at 4°C. After removal of the cornea and lens, the retinas were dissected from the posterior eye cup. Each retina was blocked for 1 hr with PBS containing 10% nonfat dried milk and 0.3% Triton-X 100 (9002-93-1; Wako), and then incubated with fluorescein isothiocyanate (FITC)-conjugated peanuts agglutinin (PNA) (1:100, L7381; Sigma-Aldrich) at 4°C overnight. Immunofluorescence images were acquired using a Fluorescence Microscope (BZ-X700; Keyence, Osaka, Japan). The numbers of PNA-positive cone cells were counted by using Image J software, ver. 1.52a (U.S. National Institutes of Health [NIH]) in 0.015625-mm² retinal areas in the superior, inferior, temporal, and nasal areas located 200, 250, 500 or 750 µm from the optic disc and each number was averaged for each area. The names and conditions of the samples were masked from the observers.

### Histologic examination

Mouse eyes were enucleated, fixed with 4% paraformaldehyde in PBS for 24 hr, and then mounted in paraffin. Sections (5 µm thick) were prepared along the horizontal meridian. The sections were subsequently stained with hematoxylin and eosin (H&E). Five sections were randomly selected from each eye. The number of cells in the outer nuclear layer (ONL) was counted in a 100 µm² square area in the central region (200 µm from the optic disc), mid-peripheral region (500 µm from the optic disc), and peripheral region (1000 µm from the optic disc) of the retina in the nasal and temporal hemispheres. The tissue samples were assigned numbers and letters, and the conditions were masked from the observers.

### TUNEL staining

TUNEL staining was performed using an ApopTag Fluorescein In Situ Apoptosis Detection Kit (Merck Millipore, Darmstadt, Germany) according to the manufacturer's instructions. Immunofluorescence images were acquired using a fluorescence microscope (BZ-X700; Keyence), and the numbers of TUNEL-positive cells in 10,000 µm² areas in the central region (200 µm from the optic nerve), mid-peripheral region (500 µm from the optic nerve), and peripheral region (1000 µm from the optic nerve) of the retina in the nasal and temporal hemispheres were counted by using Image J software, ver. 1.52a. The ONL areas in each square area were measured, and the density of TUNEL-positive cells in the ONL was calculated and expressed as cells/mm². The names and conditions of the samples were masked from the observers.

### Electroretinogram (ERG)

Photopic ERG were recorded through LED contact lenses using a PuREC system (PC-100; Mayo Corporation, Aichi, Japan). Mice were anesthetized with an intraperitoneal injection of ketamine (100 mg/ kg) and xylazine (10 mg/kg), and the body temperature was maintained at 37 °C with a heating pad. The pupils were dilated with 0.5% tropicamide and 0.5% phenylephrine hydrochloride. After topical oxbuprocaine application, LED contact lenses were attached on the mouse cornea. A reference electrode was placed to the tongue and a ground electrode was clipped to the tail. Mice were adapted for 10 min to a background of white light at an intensity of 30 cd/m². Sixteen photopic flashes were taken at 3.0 cd·s/m² and averaged.

### Preparation of PLGA nanoparticles

PLGA polymer with an average molecular weight of 20,000 and a lactide-to-glycolide copolymer ratio of 75:25 (Wako Pure Chemical Industries, Osaka, Japan) was used to prepare the nanoparticles (NP). FITC (Dojindo Laboratories, Kumamoto, Japan) or pitavastatin calcium (Wako, Osaka, Japan) (referred "pitavastatin" or "PVS" herein) was incorporated into the PLGA nanoparticles. The nanoparticles were created by using ULREA SS-11 (M Technique Co., Osaka, Japan). For FITC-NP, first, a tank containing liquid A (an aqueous solution containing 2.0% polyvinyl alcohol (PVA)) was pressurized to 0.3 MPa and then flowed at a set temperature of 43°C (measured value: approximately 40°C) and a rate of 120 ml/min. Then, liquid B (a solution containing PLGA, FITC, acetone, and ethanol in a weight ratio of 4.04 : 0.20 : 63.84 : 31.92) was flowed at a set temperature of 41°C (measured value: approximately 30°C) at 100 ml/min. The liquids A and B were reacted on a spinning disc rotating at 1,000 rpm with back pressure of 0.02 MPa. The solvent in the resultant mixture was removed by distillation using an evaporator. The resultant suspension was then purified to remove excess PVA and the unloaded reagent, and powdered by the freeze-drying method. For PVS-NP, a tank containing liquid A (an aqueous solution containing 0.17% PVA) was pressurized to 0.3 MPa and then flowed at a set temperature of 25°C (measured value: approximately 24°C) and a rate of 156 ml/min. Then, liquid B (a solution containing PLGA, PVS, acetone, and ethanol in a weight ratio of 0.7:0.15:66.10:33.05) was flowed at a set temperature of 25°C (measured value: approximately 24°C) at 100 ml/min. The liquids A and B were reacted on a spinning disc rotating at 400 rpm with back pressure of 0.02 MPa. The solvent in the resultant mixture was removed by distillation using an evaporator and the resultant was powdered by the freeze-drying method. The FITC-NP, and PVS-NP contained 6.8 ± 0.4% (w/v) FITC, and 2.79 ± 0.05% (w/v) PVS, respectively. The diameters were measured by Nanotrac Wave-EX150 (MicrotracBEL Corp). The diameters are as follows: FITC-NP, 252 nm; and PVS-NP, 202 nm.

### In vivo biodistribution of the nanoparticles

Blood samples were collected from P17 (postnatal day 17) rd10 mice 2 hr after a single intravenous injection of FITC-NP (0.5 mg FITC-NP/100 µl PBS), and the FITC incorporation in IMo was analyzed by flow cytometry. The retinas were also collected 24 hr after an intravenous injection of FITC-NP, and the FITC incorporation in macrophages and microglia was analyzed. The blood cells were labeled with mouse Ly-6C (APC-cy7-conjugated, clone HK1.4; Biolegend) and Ly-6G (PerCP/Cy5.5-conjugated, clone 1A8; Biolegend) and the following antibodies: CD192 (CCR2) (Alexa Fluor 647-conjugated, clone SA203G11; Biolegend), CD11b (BV421-conjugated, clone M1/70; Biolegend), and CX3CR1 (PE-conjugated, clone SA011F11, Biolegend). The FITC fluorescence was evaluated in CD11b⁺Ly-6C^{hi}Ly-6G^{lo-neg} IMo. For the assessment of the cellular uptake of FITC-NP, CD192 (CCR2) (FITC-conjugated, clone SA203G11; Biolegend) was not used. The retinal cells were stained with the following antibodies: CD11c (PE-Cy7-conjugated, clone N418; Biolegend), CD45 (APC-conjugated, clone 30-F11; Biolegend), Ly-6C (APC-cy7-conjugated, clone HK1.4; Biolegend), Ly-6G (APC-cy7-conjugated, clone 1A8; Biolegend), CD11b (PE-conjugated, clone M1/70, Biolegend), and CX3CR1 (BV421-conjugated, clone SA011F11; Biolegend). The fluorescence of FITC in microglia and macrophages was then measured.

### NP treatment of rd10 mice

We divided rd10 mice into three groups at P21: the PBS group (100 µl PBS), the FITC-NP group (0.5 mg FITC-NP/100 µl PBS), and the PVS-NP group (0.0065 mg PVS/0.5 mg PVS-NP/100 µl PBS; equivalent to 0.325 mg PVS/kg body weight). PBS, FITC-NP, or PVS-NP was administered intravenously via the tail vein twice a week (once every three days or four days) from P21 until the end of each experiment.

### Dose-finding study

To determine the optimal dosage of PVS-NP, we divided rd10 mice into four groups at P21 to perform a dose-finding study: the PBS group (100 µl PBS), the PVS-NP low group (0.1 mg PVS/kg), the PVS-NP middle group (0.3 mg PVS/kg), and the PVS-NP high group (1.0 mg PVS/kg). PBS or PVS-NP was administered intravenously via the tail vein once a week from P21 until the end of each experiment. Analyses for the photopic ERG and the number of PNA-positive cone cells were performed at P49.

### Schedule-finding study

To determine the optimal administration schedule of PVS-NP, we divided rd10 mice into three groups at P21 to perform a schedule-finding study: the PBS (100 µl PBS) every two weeks group, the PVS-NP (0.75 mg PVS/kg) every four weeks group, and the PVS-NP (0.5 mg PVS/kg) every two weeks group. PBS or PVS-NP was administered intravenously via the tail vein for each administration period from P21 until the end of each experiment. Analyses for the photopic ERG and the number of PNA-positive cone cells were performed at P49.

### Statistical analyses

The correlation coefficients between the MD slope in the RP patients' HFA10-2 tests and the percentage of monocyte subsets in the patients were analyzed by Spearman's rank correlation test. Comparisons of the data between pairs of groups were performed using the Wilcoxon rank sum test. A p-value ≤0.05 was accepted as significant. The statistical analyses of the data were performed with JMP1 Pro 13.0.0 software (SAS, Cary, NC).

### 2. Results

### Increased inflammatory monocytes in the peripheral blood of rd10 mice and RP patients

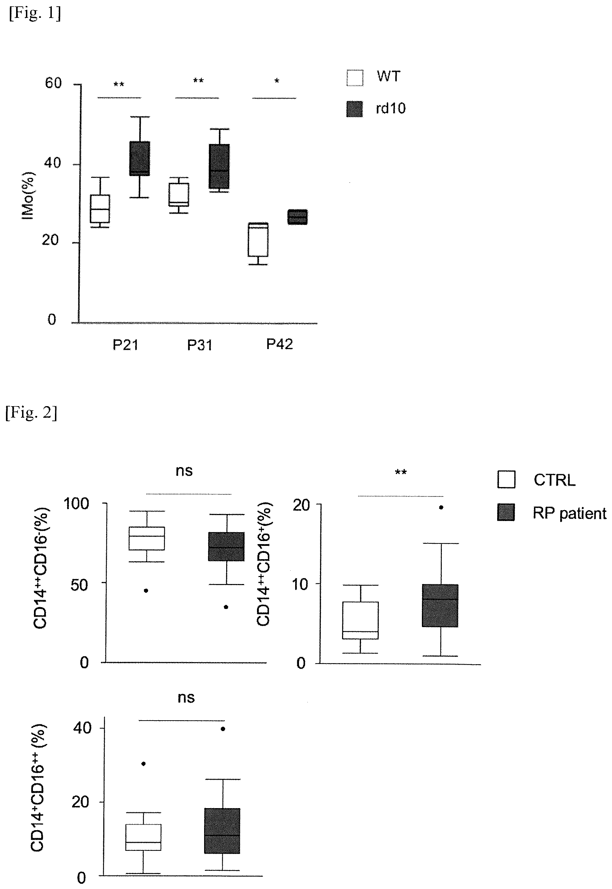

To investigate whether RP is associated with increase in the number of circulating monocytes, we analyzed the number of inflammatory monocytes (IMo) in the blood of rd10 mice, a clinically relevant model of RP with Pde6b mutation. In rd10 mice, rod cell death starts around P18 and most rod cells disappear by P30; this is followed by gradual cone degeneration. The peripheral blood of wildtype (WT) and rd10 mice was analyzed by flow cytometry at P21, P31, and P42. The results demonstrated significant increase in the number of CD11b⁺Ly-6C^{hi}Ly-6G^{lo} IMo in the rd10 mice at P21 (p<0.01), P31 (p<0.01) and P42 (p<0.05) compared with that of the WT mice (Fig. 1). These cells also expressed high levels of CCR2 and CX3CR1 (data not provided).

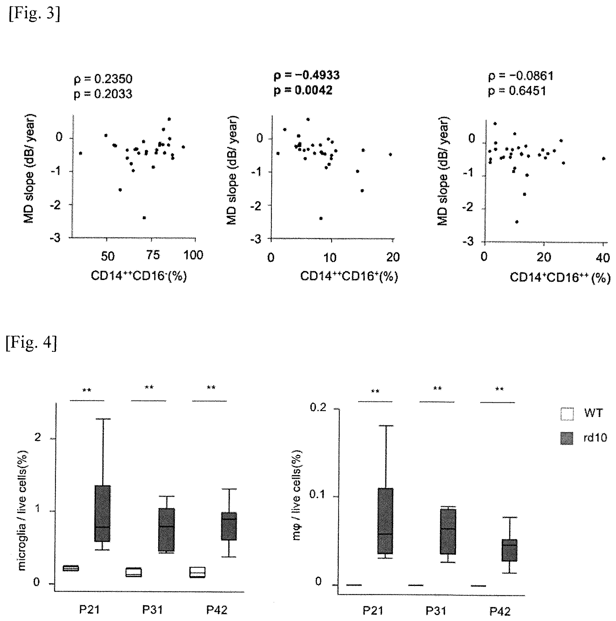

We next evaluated monocyte changes in the RP patients. The subjects' peripheral blood samples were analyzed by flow cytometry, and monocytes were classified into CD14⁺CD16⁺⁺ non-classical, CD14⁺⁺CD16⁺ intermediate, and CD14⁺⁺CD16⁻ classical monocytes. The results revealed no significant difference in the proportion of total monocytes between the RP patients and controls, but subset analysis showed significant increase in the percentage of CD14⁺⁺CD16⁺ intermediate monocytes in RP patients (p=0.0098; Fig. 2), which also co-expressed high levels of CCR2 and CX3CR1 (data not provided). In addition, we found a correlation between the higher percentage of CD14⁺⁺CD16⁺ intermediate monocyte subset and greater MD slope (decline rate of retinal sensitivity) (p=-0.4933, p=0.0042; Fig. 3). These findings suggest that RP is associated with increase in peripheral blood IMo in both humans and animal models of the disease.

### Changes of retinal microglia and macrophages in rd10 mice

To investigate the recruitment of IMo into the retina of rd10 mice, we performed flow cytometry analysis of the retinal myeloid cells. Retinal microglia and macrophages can be distinguished by using surface markers CD45 and CD11c. Accordingly, we stained retinal cells with CD11b, CD11c, CD45, Ly6C, Ly6G, CCR2, and CX3CR1. In WT mice, CD11b^{hi}CD11c^{mid}CD45^{mid}Ly-6G^{lo}Ly-6C^{lo} microglia, but not CD11b^{hi}CD11c^{hi}CD45^{hi}Ly-6G^{lo}Ly-6C^{lo} macrophages, were present in the retina. In contrast, the rd10 mice retina had increased numbers of both cell populations at P21, P31, and P42 (p<0.01; Fig. 4).

### Association between the CCL2/CCR2 axis and cone cell death in rd10 mice

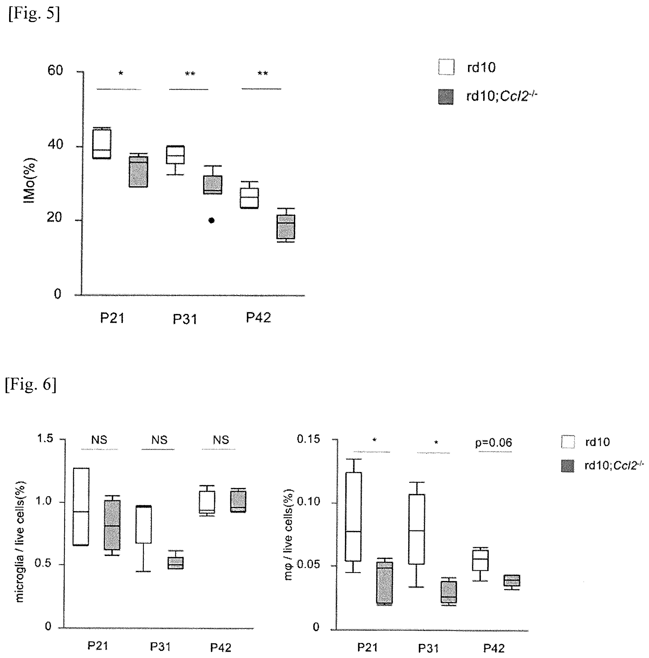

To investigate the roles of IMo and peripherally-derived macrophages, we generated rd10 mice that were deficient for Ccl2 (rd10;Ccl2^{-/-}), wherein the CCL2/CCR2 axis, which is essential for recruiting bone marrow cells into the blood and diseased foci, is blocked. Ccl2 deficiency decreased the population of peripheral IMo at P21, P31, and P42 (p<0.05, p<0.01, and p<0.01 respectively; Fig. 5). In the retinas of rd10;Ccl2^{-/-} mice, macrophages were significantly decreased at P21 (p<0.05) and at P31 (p<0.05) and marginally decreased at P42 (p=0.06), but the proportions of resident microglia were not significantly different between rd10;Ccl2^{-/-} and rd10;Ccl2^{+/+} mice (Fig. 6). These data show that the CCL2/CCR2 axis is important for IMo/macrophages recruitment in rd10 mice.

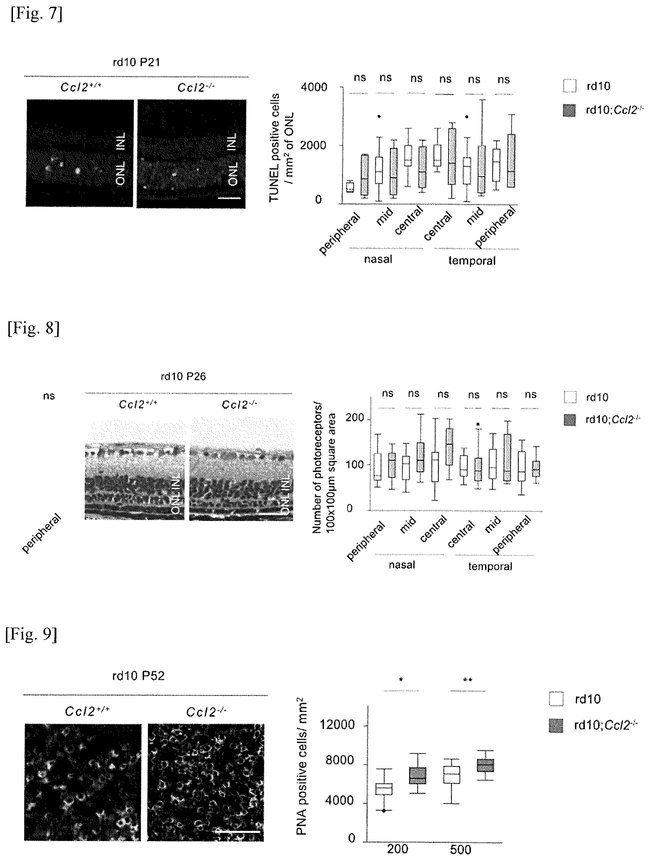

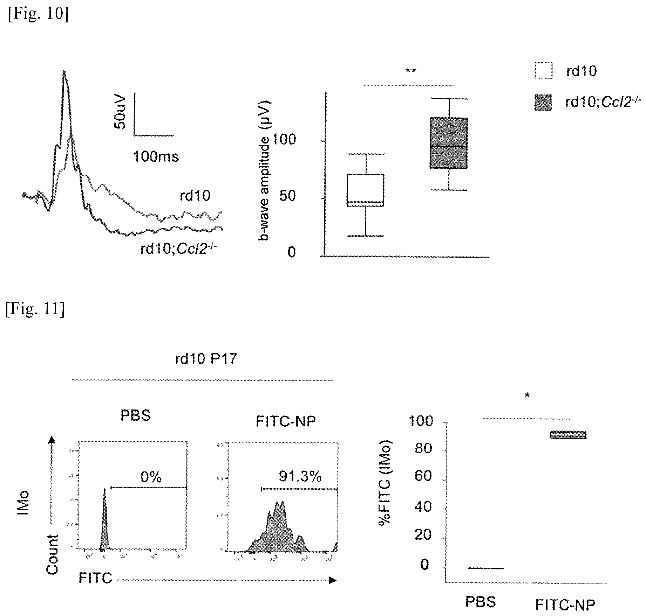

We next evaluated the effect of Ccl2 deficiency on rod and cone degeneration in rd10 mice. TUNEL staining at P21, when the rod cell death peaked, showed no significant difference in the number of TUNEL-positive cells in the outer nuclear layer (ONL) between rd10;Ccl2^{-/-} and rd10;Ccl2^{+/+} mice (Fig. 7). Consistently, the HE staining at P26 demonstrated that there was no significant difference in ONL thickness in the presence or absence of Ccl2 (Fig. 8), suggesting that Ccl2 deficiency may not influence rod degeneration in rd10 mice. In contrast, the cone cell density, as assessed with peanut agglutinin (PNA) labeling, was significantly higher in the P52 rd10;Ccl2^{-/-} mice compared with the rd10;Ccl2^{+/+} mice (Fig. 9). Furthermore, we analyzed cone function by photopic ERG. The photopic ERG b-wave was significantly maintained in rd10;Cc12^{-/-} mice compared with rd10 mice (Fig. 10). These results suggest that the CCL2/CCR2 axis facilitates recruitment and engraftment of peripheral macrophages into the retina, and this contributes to cone degeneration in rd10 mice.

### Drug delivery to peripheral blood IMo and retinal macrophages

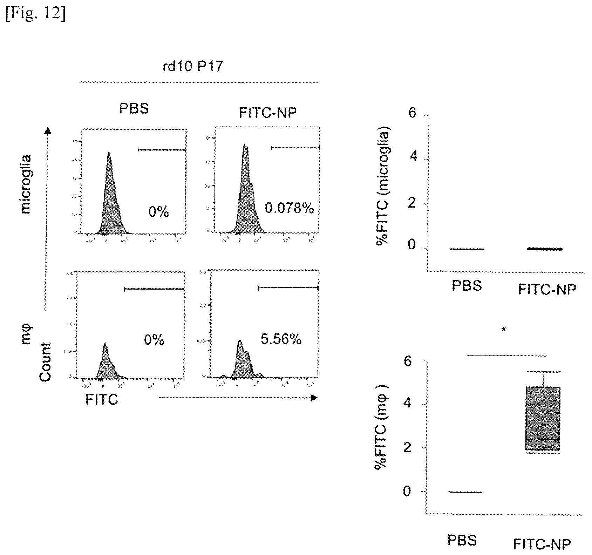

We investigated drug delivery to peripheral blood IMo and peripherally-derived macrophages by PLGA nanoparticles. The specificity of the delivery was evaluated by injecting FITC-loaded nanoparticles to rd10 mice via the tail vein and analyzing the delivery of FITC in Ly-6C^{hi} IMo 2hr after the injection. FITC-NP efficiently delivered FITC to a large number of IMo (p<0.05; Fig. 11). The drug delivery efficiency to retinal microglia and macrophages was analyzed 24 hr after the intravenous injection of the nanoparticles. By the administration of FITC-NP, introduction of FITC was observed in 3.1 ± 1.7 % of macrophages. In contrast, no FITC was detected in microglia after the administration of FITC-NP (Fig. 12). These results suggest that the nanoparticles are a promising drug delivery system that targets peripherally-derived macrophages but not microglia.

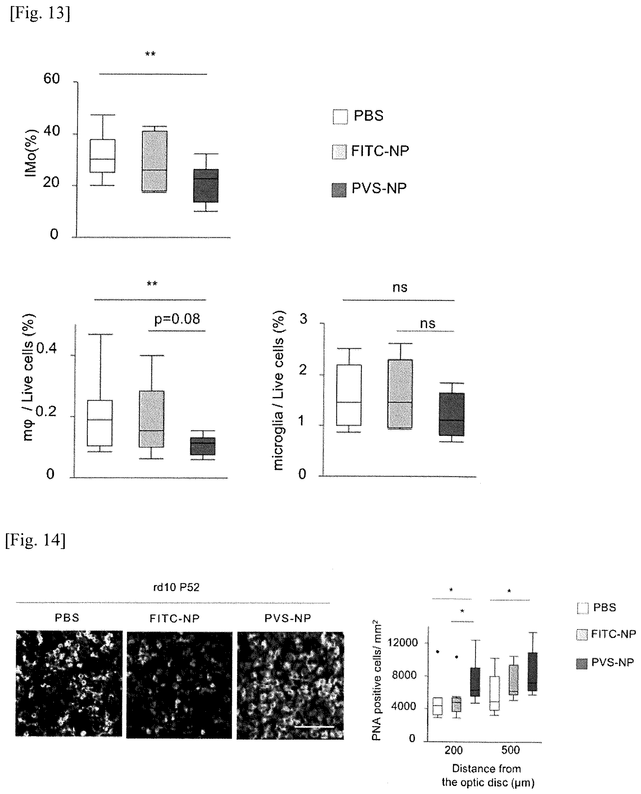

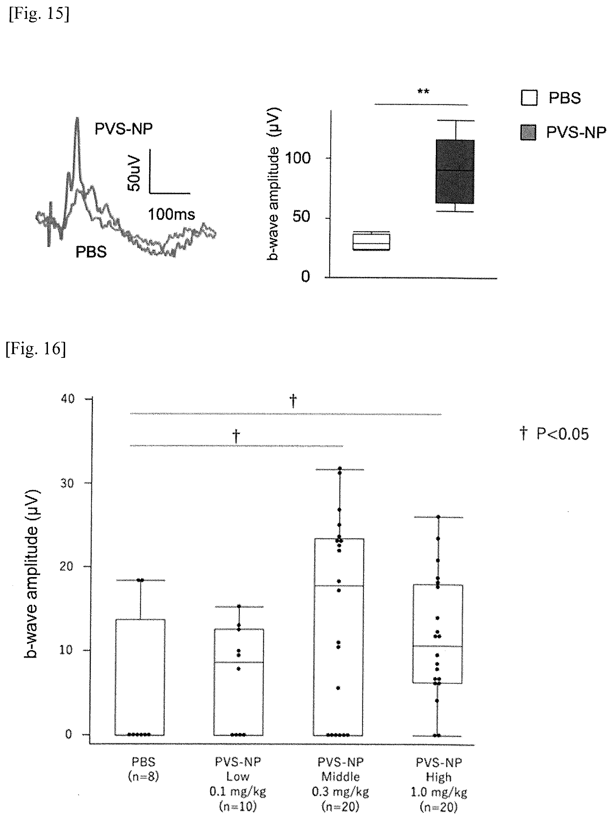

Using the drug delivery system, the efficacy of pitavastatin-loaded nanoparticles (PVS-NP) was evaluated in rd10 mice. The rd10 mice were intravenously injected with PBS, FITC-NP, or PVS-NP twice a week from P21. At P31, the numbers of blood IMo and retinal macrophages were significantly decreased in the PVS-NP group compared to the PBS group (Fig. 13). There was no significant difference in the percentage of microglia between the three groups (Fig. 13). At P52, the cone cell density was significantly maintained in the PVS-NP group compared to the PBS and FITC-NP groups (Fig. 14). At P35, the photopic ERG wave was significantly maintained in the PVS-NP group compared to the PBS group (Fig. 15).

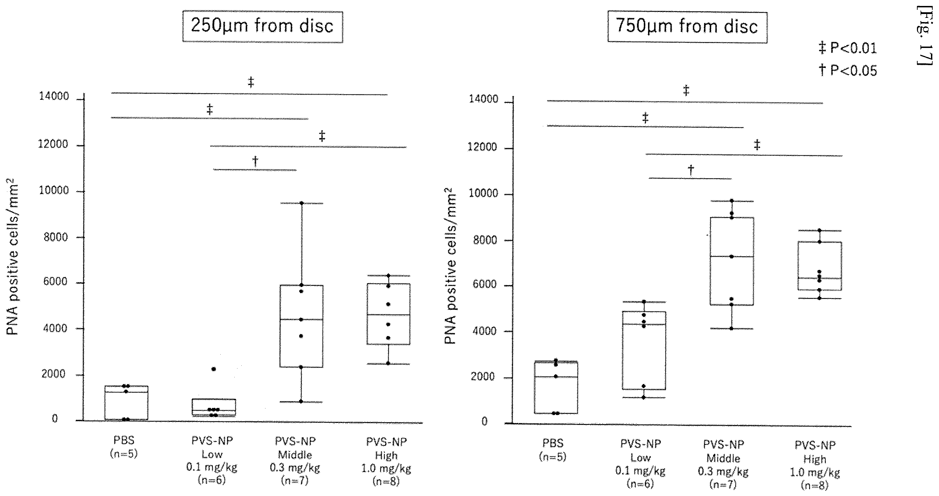

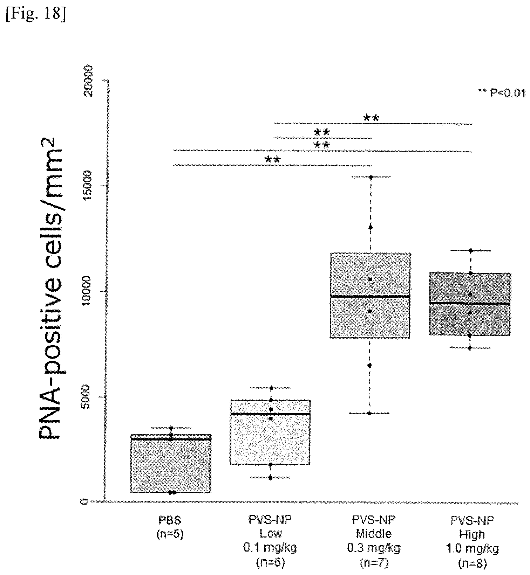

To determine the optimal dosage of PVS-NP, we divided rd10 mice into four groups at P21 to perform a dose-finding study: the PBS group (100 µl PBS), the PVS-NP low group (0.1 mg PVS/kg), the PVS-NP middle group (0.3 mg PVS/kg), and the PVS-NP high group (1.0 mg PVS/kg). PBS or PVS-NP was administered intravenously via the tail vein once a week from P21 until the end of each experiment. Analyses for the photopic ERG and the number of PNA-positive cone cells were performed at P49. The photopic ERG b-wave was significantly higher in the PVS-NP middle and PVS-NP high groups compared to the PBS group (p<0.05; Fig. 16). For the number of PNA-positive cone cells, similarly, the cone degeneration was significantly suppressed in the PVS-NP middle and PVS-NP high groups compared to the PBS group (p<0.01; Figs. 17 and 18).

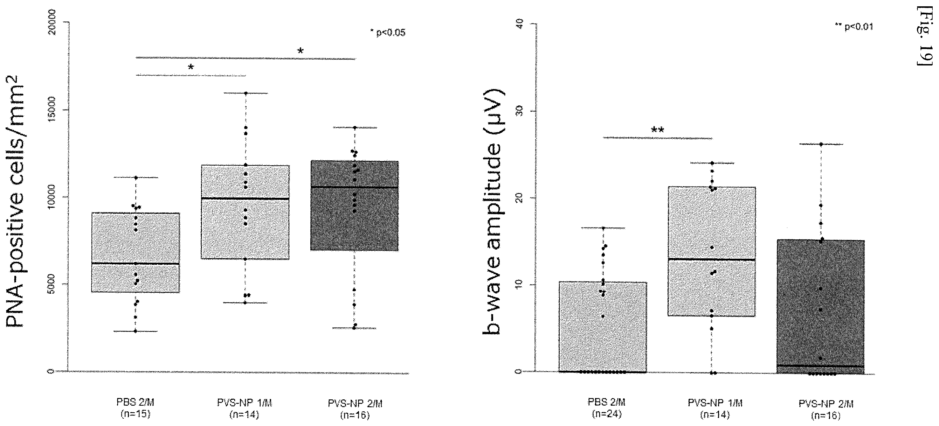

To determine the optimal administration schedule of PVS-NP, we divided rd10 mice into three groups at P21 to perform a schedule-finding study: the PBS (100 µl PBS) every two weeks group, the PVS-NP (0.75 mg PVS/kg) every four weeks group, and the PVS-NP (0.5 mg PVS/kg) every two weeks group. PBS or PVS-NP was administered intravenously via the tail vein for each administration period from P21 until the end of each experiment. Analyses for the photopic ERG and the number of PNA-positive cone cells were performed at P49. The photopic ERG b-wave was significantly higher in the PVS-NP (0.75 mg PVS/kg) every four weeks group compared to the PBS group (p<0.01; Fig. 19). For the number of PNA-positive cone cells, the cone degeneration was significantly suppressed in the PVS-NP (0.75 mg PVS/kg) every four weeks group and PVS-NP (0.5 mg PVS/kg) every two weeks group compared to the PBS group (p<0.05; Fig. 19).

These results demonstrate that the statin-loaded nanoparticles are a promising therapeutic option for retinitis pigmentosa.

## Claims

1. A pharmaceutical composition for use in treating retinitis pigmentosa comprising statin-loaded nanoparticles.

2. The pharmaceutical composition for use according to claim 1, wherein the statin is pitavastatin or a pharmaceutically acceptable salt thereof.

3. The pharmaceutical composition for use according to claim 1 or 2, wherein the statin is pitavastatin calcium.

4. The pharmaceutical composition for use according to any one of claims 1 to 3, wherein the statin-loaded nanoparticles contain 1 to 15% by weight of statin.

5. The pharmaceutical composition for use according to any one of claims 1 to 4, wherein the statin-loaded nanoparticles comprise PLGA.

6. The pharmaceutical composition for use according to any one of claims 1 to 5, wherein the statin-loaded nanoparticles have a particle size of 50 to 300 nm.

7. The pharmaceutical composition for use according to any one of claims 1 to 6, wherein the pharmaceutical composition comprises 1 to 15% by weight of the statin-loaded nanoparticles.

8. The pharmaceutical composition for use according to any one of claims 1 to 7, wherein the pharmaceutical composition is administered at a dose of 0.01 mg/kg to 0.5 mg/kg per day of statin.

9. The pharmaceutical composition for use according to any one of claims 1 to 8, wherein the pharmaceutical composition is administered intravenously.

## Patentansprüche

1. Eine pharmazeutische Zusammensetzung zur Verwendung bei der Behandlung von Retinitis pigmentosa, umfassend mit Statin beladene Nanopartikel.

2. Die pharmazeutische Zusammensetzung zur Verwendung gemäß Anspruch 1, wobei das Statin Pitavastatin oder ein pharmazeutisch verträgliches Salz davon ist.

3. Die pharmazeutische Zusammensetzung zur Verwendung gemäß Anspruch 1 oder 2, wobei das Statin Pitavastatin-Calcium ist.

4. Die pharmazeutische Zusammensetzung zur Verwendung gemäß einem der Ansprüche 1 bis 3, wobei die mit Statin beladenen Nanopartikel 1 bis 15 Gew.-% Statin enthalten.

5. Die pharmazeutische Zusammensetzung zur Verwendung gemäß einem der Ansprüche 1 bis 4, wobei die mit Statin beladenen Nanopartikel PLGA umfassen.

6. Die pharmazeutische Zusammensetzung zur Verwendung gemäß einem der Ansprüche 1 bis 5, wobei die mit Statin beladenen Nanopartikel eine Teilchengröße von 50 bis 300 nm aufweisen.

7. Die pharmazeutische Zusammensetzung zur Verwendung gemäß einem der Ansprüche 1 bis 6, wobei die pharmazeutische Zusammensetzung 1 bis 15 Gew.-% der mit Statin beladenen Nanopartikel umfasst.

8. Die pharmazeutische Zusammensetzung zur Verwendung gemäß einem der Ansprüche 1 bis 7, wobei die pharmazeutische Zusammensetzung in einer Dosis von 0,01 mg/kg bis 0,5 mg/kg Statin pro Tag verabreicht wird.

9. Die pharmazeutische Zusammensetzung zur Verwendung gemäß einem der Ansprüche 1 bis 8, wobei die pharmazeutische Zusammensetzung intravenös verabreicht wird.

## Revendications

1. Composition pharmaceutique pour l'utilisation dans le traitement de la rétinite pigmentaire comprenant des nanoparticules chargées de statine.

2. Composition pharmaceutique pour l'utilisation selon la revendication 1, dans laquelle la statine est la pitavastatine ou un sel pharmaceutiquement acceptable de celle-ci.

3. Composition pharmaceutique pour l'utilisation selon la revendication 1 ou 2, dans laquelle la statine est la pitavastatine calcique.

4. Composition pharmaceutique pour l'utilisation selon l'une quelconque des revendications 1 à 3, dans laquelle les nanoparticules chargées de statine contiennent 1 à 15 % en poids de statine.

5. Composition pharmaceutique pour l'utilisation selon l'une quelconque des revendications 1 à 4, dans laquelle les nanoparticules chargées de statine comprennent du PLGA.

6. Composition pharmaceutique pour l'utilisation selon l'une quelconque des revendications 1 à 5, dans laquelle les nanoparticules chargées de statine ont une taille de particules de 50 à 300 nm.

7. Composition pharmaceutique pour l'utilisation selon l'une quelconque des revendications 1 à 6, la composition pharmaceutique comprenant 1 à 15 % en poids des nanoparticules chargées de statine.

8. Composition pharmaceutique pour l'utilisation selon l'une quelconque des revendications 1 à 7, la composition pharmaceutique étant administrée à une dose de 0,01 mg/kg à 0,5 mg/kg par jour de statine.

9. Composition pharmaceutique pour l'utilisation selon l'une quelconque des revendications 1 à 8, la composition pharmaceutique étant administrée en intraveineuse.
